# EUROPEAN PATENT SPECIFICATION

(11) **EP 1 644 733 B1**
(45) Date of publication and mention of the grant of the patent: **11.04.2007**
(21) Application number: 05708384.2
(22) Date of filing: 17.02.2005
(51) Int. Cl.: G01N 33/50, A01K 67/027

(54) **SCREENING METHODS EMPLOYING ZEBRAFISH AND THE BLOOD BRAIN BARRIER**
SCREENING-VERFAHREN UNTER VERWENDUNG VON ZEBRAFISCH UND DER BLUT-HIRN-SCHRANKE
PROCEDES DE CRIBLAGE METTANT EN OEUVRE LE POISSON ZEBRE ET LA BARRIERE HEMATO-ENCEPHALIQUE

(30) Priority: 17.02.2004 GB 0403490
(43) Date of publication of application: 12.04.2006
(73) Proprietor: Daniolabs Limited, Cambridge, Cambridgeshire CB5 9TN (GB)
(72) Inventor: GOLDSMITH, Paul, Daniolabs Limited, Cambridge, Cambridgeshire CB5 9TN (GB); FLEMING, Angeleen, Louise, Daniolabs Limited, Cambridge Cambridgeshire CB5 9TN (GB)
(74) Representative: Walton, Seán Malcolm
(86) International application number: PCT/GB2005/000583
(87) International publication number: WO 2005/080974

(56) References cited:
- KHAN NAIM A ET AL: "Role of serotonin in fish immunomodulation" JOURNAL OF EXPERIMENTAL BIOLOGY, vol. 200, no. 13, 1997, pages 1833-1838, XP002327894 ISSN: 0022-0949
- PARDRIDGE WILLIAM M: "Drug and gene delivery to the brain: The vascular route." NEURON, vol. 36, no. 4, 14 November 2002 (2002-11-14), pages 555-558, XP002327895 ISSN: 0896-6273
- PETERSON R T: "Discovery of therapeutic targets by phenotype-based zebrafish screens" DRUG DISCOVERY TODAY: TECHNOLOGIES, ELSEVIER, vol. 1, no. 1, September 2004 (2004-09), pages 49-54, XP004767913 ISSN: 1740-6749

## Description

The present invention relates to use of fish, in particular zebrafish, in screening for substances with an ophthalmological effect or biological effect on the brain or central nervous system and/or effect on a disease or disorder of the brain or central nervous system. It further relates to use of zebrafish in screening for substances that have a desired biological activity and which do not cross the blood brain barrier.

The invention is based in part on the inventors' finding that zebrafish have a blood brain barrier (BBB) and that this blood brain barrier becomes established at defined times. While all vertebrates have some form of BBB, this barrier is poorly characterised in lower vertebrates. While some clear differences have been identified in the properties of the BBB in teleosts and mammals, e.g. monoamines can cross the BBB of teleosts but not rodents (Khan and Deschaux, 1997) little is known about how the BBB differs in terms of either structure or function between vertebrate species. Furthermore, it is known that, in mammals, the BBB "tightens" throughout development and is only mature in post-natal animals (Ibiwoye et al., 1994; Wolburg and Lippoldt, 2002; Nag., 2003). One of the major benefits of the zebrafish as a model organism is the ability to perform screens on juvenile stages, but knowledge about the presence of a blood brain barrier in such organisms and developmental time of formation is not available in the art. The knowledge now provided for the first time herein has important technical application in design and execution of screens for substances that have desired biological effects in the brain or central nervous system, ophthalmologically and so on.

In mammals, the BBB provides a complex barrier to the penetration of drugs into the CNS. The capillary network in the brain is so dense that every neuron and glial cell is no more than 20 micrometers from a neighbouring capillary. Yet the presence of the blood-brain barrier has been one of the greatest obstacles in the development of drugs to treat neurological conditions, with less than 1% of small molecules penetrating this barrier. There are additionally arachnoid epithelial membrane barriers and choroid plexus epithelial barriers, although the blood-brain barrier has 1000 fold greater surface area than the blood-CSF and arachnoid membrane barriers and therefore is quantitatively the most important barrier system (Dohrmann, 1970).

The first level of barrier is presented by the endothelial tight junctions, which possess the high resistance tight junctions seen in epithelial cells, rendering brain capillary endothelia sealed, unlike their leaky peripheral endothelial cousins. There is thus no paracellular movement of fluid and only minimal pinocytosis (Brightman, 1977). The three component cells of the blood-brain barrier are the endothelial cells themselves, the capillary pericytes and the perivascular astrocyte foot processes (Pardridge, 2003), all of which express a variety of enzymes such as aminopeptidases, carboxypeptidases, endopeptidases and cholinesterases which inactivate many drugs (Pardridge, 2002), although the enzymes may also activate pro-drugs. Transporter systems permit the entry of a variety of molecules that would otherwise not enter the brain (Pardridge, 2002). Additionally, certain molecules freely diffuse across the blood-brain barrier, but are actively effluxed by active efflux transporters, the most notable of which is p-glycoprotein (pgp) (Tsuji and Tamai, 1999).

It has not previously been established whether zebrafish have a blood-brain barrier at all. There is evidence of some sort of BBB in a number of species of fish, but there have been no previous reports of a BBB in zebrafish. (Borg-Neczak and Tjalve, 1996) looked at pike and showed selective uptake of peripherally injected mercury in adult trout, suggesting exclusion across most of the brain, implying a blood-brain barrier. A further study on rainbow trout (42 days old), using 3-OMG, a non-metabolizable glucose analogue that uses the same transport carrier as glucose presented results indicative of facilitated uptake into the brain, similar to that also reported for tryptophan in rainbow trout brain (Aldegunde et al., 2000), although the time course of radioactivity levels in brain and plasma could be explained by an incomplete blood-brain barrier. A similar result in rainbow trout showing axonal transport bypassing a blood-brain barrier was reported for tributyltin, a waterborne organometal (Rouleau, Xiong, Pace Pavicius 2003). There have, however, been no reports to date on whether zebrafish possess a BBB and indeed there is also contradictory evidence suggesting certain fish species do not have a blood brain barrier. Bachaur (Bachaur, Failing, Georgii) examined concentrations of polychlorinated biphenyls (PCBs) in various tissues of rainbow trout (of unstated ages), foxes, roe deer and humans and noted exclusion of PCBs from the brain of mammals in comparison to fish, concluding that the blood-brain barrier in fish must be less efficient than in mammals. Khan and Deschaux (Journal of Experimental Biology, Volume 200, 1997) noted that biogenic amines could pass through the blood-brain barrier of teleost fish, referring to the evidence of Fritsche (Fritsche, Reid, Thomas and Perry, 1993), although the referred to paper does not appear to provide any evidence for this.

A further unknown in the art beyond whether zebrafish have a blood-brain barrier, is how mature is any such a blood-brain barrier in relation to a more advanced organism. In mammals the effective brain concentration of a compound is profoundly dependent on active influx and efflux pumps, most notably pgp (Schumacher and Mollgard, 1997). Pore size is also under tight regulatory control involving molecules such as claudin-5 (Nitta et al., 2003).

Furthermore, it is recognised that the blood-brain barrier gradually matures during development in mammals, with permeability to small molecules decreasing with age. It is known that substances excluded from the adult rat brain do permeate embryonic brain capillaries (Wolburg and Lippoldt, 2002). Similarly the developing mouse BBB shows decreased permeability to compounds as it matures (Stewart and Hayakawa, 1987).

These contribution made by the work described herein is critical in the interpretation of potential blood brain barrier penetration in zebrafish, given that the evolutionary strategy of zebrafish is to develop extremely rapidly to attain an adult-like stage within 72 hours in order to be able to escape predation. Thus by day four they are already able to see, escape predation, and feed, although sexual maturity is not reached until three months. This rapid development has enabled many processes to be studied and modelled at this larval stage when their small size and easy husbandry makes them most suitable for compound screening. It has previously been unknown, however, as to the sophistication of any blood brain barrier development in zebrafish and how mature any such barrier is at the larval stages.

It is shown herein that zebrafish do indeed develop a sophisticated blood-brain barrier with active transport systems and that it develops in a time-dependent manner. The present invention is thus able to provide for the first time rational strategies for screening compounds for neurological indications, as well as generating an *in vivo* system for determining a compound's brain penetration *in vivo*.

### Brief Description of the Figures

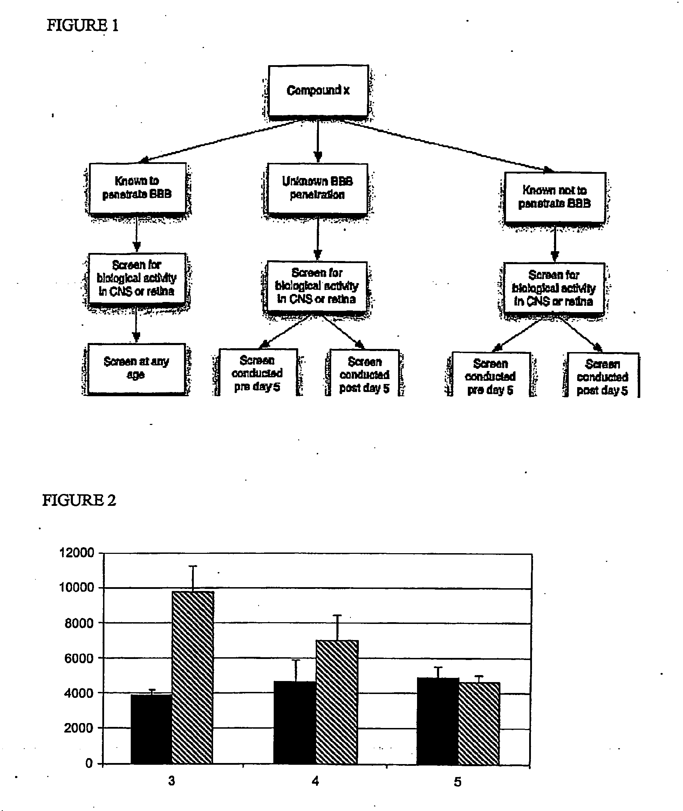

Figure 1 is a schematic flowchart setting out screening in accordance with an embodiment of the present invention. Here, as elsewhere here, reference to "post day 5" is to 5 days post-fertilisation or later, i.e. following establishment of the blood brain barrier as determined herein.
Figure 2 is a histogram showing mean fluorescent intensity in the brain of Evans Blue injected fish and saline controls. The X-axis displays days post-fertilisation and the Y axis displays fluorescent intensity in the brain. Each set of bars represents a single time point with solid bars representing saline injected controls and shaded bars representing Evans Blue injected samples. There is an age dependent decrease in the fluorescent intensity in the brain, indicative of the exclusion of Evans Blue as the BBB matures.

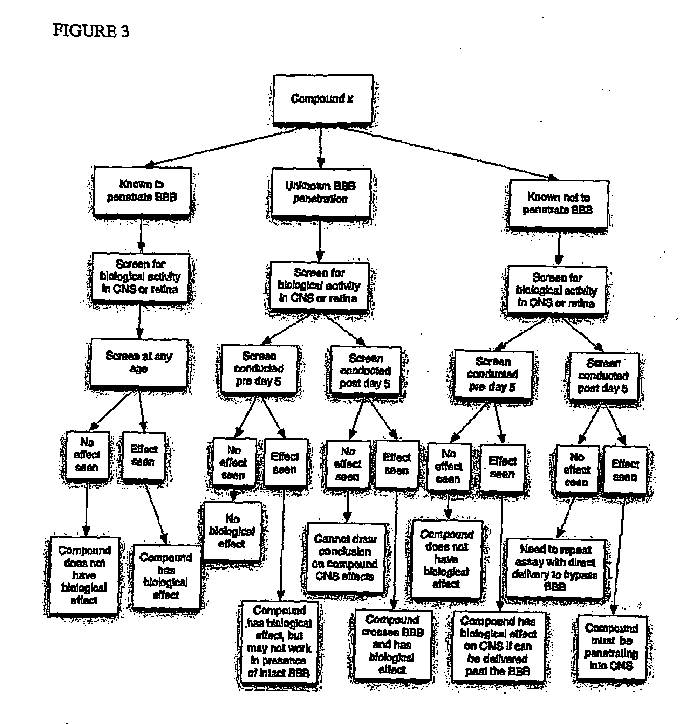

Figure 3 is a schematic flowchart incorporating the features of claim 1 and further illustrating use of results obtained in screens according to the present invention in assessing biological activities of test substances.

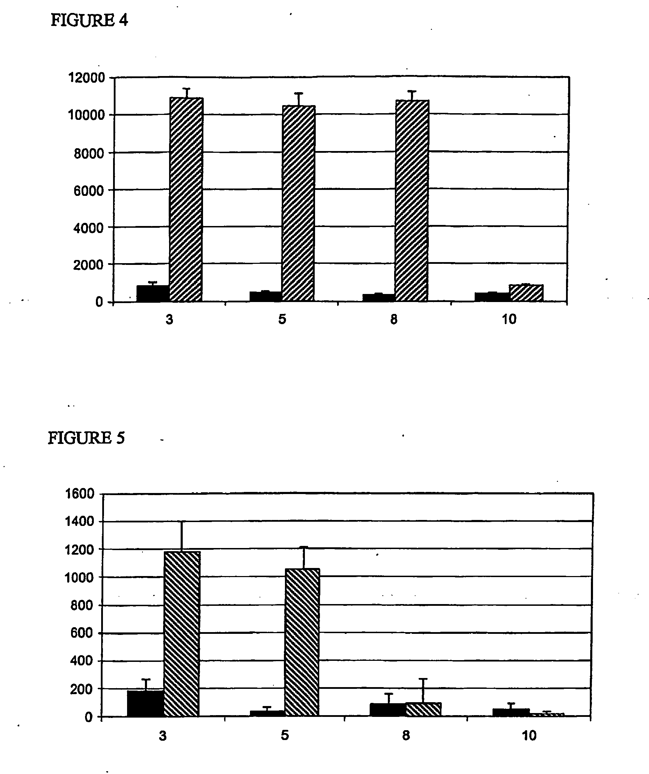

Figure 4 shows the exclusion of sodium fluorescein dye, a low molecular weight dye, from the brain by 10 days post-fertilisation (d.p.f.). The X axis displays days post-fertilisation and the Y axis displays fluorescent intensity in the brain. Each set of bars represents a single time point with solid bars representing saline injected controls and shaded bars representing sodium fluorescein injected samples. There is a sharp age dependent decrease in the fluorescent intensity in the brain at 10 d.p.f., indicative of the exclusion of small molecules from the brain at this time.
Figure 5 shows the exclusion of rhodamine 123 (R123) dye, a low molecular weight dye, from the brain by 10 days post-fertilisation (d.p.f.). Although similar in molecular weight to sodium fluorescein, R123 is a substrate for Pgp and is therefore actively transported out of the CNS. The X axis displays days post-fertilisation and the Y axis displays fluorescent intensity in the brain. Each set of bars represents a single time point with solid bars representing saline injected controls and shaded bars representing R123 injected samples. There is a sharp age dependent decrease in the fluorescent intensity in the brain at 8 d.p.f., indicative of the exclusion of R123 from the brain at this time. This time point coincides with the onset of expression of Pgp in the vascular endothelium of the CNS. Hence, although sodium fluorescein and R123 are of similar size, R123 is excluded from the brain at younger ages than sodium fluorescein as it is actively removed by Pgp.

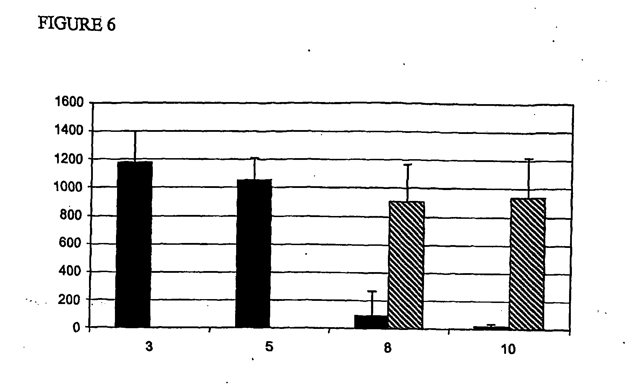

Figure 6 shows that exclusion of rhodamine 123 (R123) dye from the brain is through a Pgp-dependent mechanism. The.X axis displays days post-fertilisation and the Y axis displays fluorescent intensity in the brain. Each set of bars represents a single time point with solid bars representing R123 injected larvae raised in embryo medium and shaded bars representing R123 injected samples raised in the presence of a Pgp inhibitor, verapamil (V). When raised in normal conditions (embryo medium), there is a sharp age dependent decrease in the fluorescent intensity in the brain at 8 d.p.f., indicative of the exclusion of R123 from the brain at this time and coincident with the expression of Pgp as detected by immunohistochemistry. This age-dependent exclusion is reversed by incubating larvae in embryo medium containing the Pgp inhibitor verapamil such that fluorescence in the brains of these samples at 8 d.p.f. and 10 d.p.f. is equivalent to that observed at younger ages (3 and 5 d.p.f.).

It is well known that pharmaceutical research leading to the identification of a new drug may involve the screening of very large numbers of candidate substances, both before and even after a lead compound has been found. This is one factor which makes pharmaceutical research very expensive and time-consuming. Means for assisting in the screening process have considerable commercial importance and utility.

Zebrafish are becoming increasingly popular as a sophisticated screening tool for the effect of small molecules on complex physiological and pathological processes (Goldsmith, 2004). This is because they are vertebrates with a similar genome size and complexity to humans, yet it is possible to generate hundreds of thousands of offspring in a moderate size of aquarium every year. As the offspring are no more than a few millimetres in length, with larvae living in volumes as small as 50 microliters, they are particularly amenable to high-throughput compound screening. An increasing number of models of human disease are being reported in the literature, such as for Parkinson's disease (Anichtchik et al., 2004), retinal degenerations (Goldsmith et al., 2003), and sensorineural deafness (Whitfield, 2002).

Whilst the effects of a number of pharmaceutical compounds have been shown to be similar in zebrafish to mammals (Langheinrich, 2003), a major question regarding the utility of small molecule screening for a neurological or ophthalmological condition is whether a small molecule will reach its target site. As noted, the present invention provides an important contribution to the design and use of new screens and assay methods, allowing for identification and use of new drugs.

In various further aspects the present invention relates to screening and assay methods and means, and substances identified thereby.

More specifically, the present invention employs zebrafish in screening and assay methods for substances that are biologically active in the brain or central nervous system (CNS) and/or exert an ophthalmological biological effect, e.g. in ameliorating one or more symptoms of a disease or disorder of the brain or central nervous system, wherein the screening or assay methodology takes into account knowledge (1) that zebrafish have a blood brain barrier and (2) that the blood brain barrier forms in zebrafish embryos at five days.

It should be noted that reference herein to establishment of a blood brain barrier at five days post fertilisation is, a reference to establishment of a blood brain barrier sufficient to exclude molecules of comparable Mr to Evans Blue. For smaller molecules, effective blood brain barrier development may be excluded later. Thus, in all aspects and embodiments of the present invention, should it be determined that smaller molecules are able to pass the blood brain barrier at five days post fertilisation but are excluded from a later point in time, then that point in time (e.g. 6 days or 7 days post fertilisation) is to be substituted for reference to 5 days post fertilisation where the test substance is of such smaller size.

More specifically, it is established herein that molecules of 960 daltons or greater are excluded at five days and molecules of less than 960 daltons are excluded at ten days, except where the is active efflux of the molecules by pgp at eight days, the stated ages refering to zebrafish grown under standard conditions and are adjusted if the zebrafish are grown under conditions that accelerate or retard achievement of the equivalent stage of development.

As any zebrafish biologist will be aware, there may be slower or faster development rates if in particular the temperature of the aquarium is altered or if a different strain is used. Increased temperature is known to accelerate development while lower temperature retards development. Standard conditions involve growing zebrafish at a temperature of 28.5 °C. More specifically, conditions may be those defined by Westerfield (1995) and further defined by Kimmel et al., (1995), specifically a temperature of 28.5 °C at low density (no more than 5 larvae per ml) in embryo medium (5 mM NaCl, 0.17 mM KCl,0.33 mMCaCl₂, 0.33 mM Mg₂SO₄, 10⁻⁵% Methylene Blue) on a 14 hour day, 10 hour night light cycle.

Under such conditions the following developmental stages are reached at 5, 8 and 10 days respectively:
(i) 5 days - the time at which goblet cells first present in the posterior gut, ossification is present in the operculum and the sheath of the anterior tip of the notochord;
(ii) 8 days - the time at which ossification of the centra is evident in the vertebral column, Pgp is expressed in the vascular endothelium surrounding the CNS and mature compacted myelin is seen in the hindbrain;
(iii) 10 days - the time at which ossification centres are evident in Meckels cartilage and the palatoquadrate, neural arches are present on cervical vertebrae, ossification is apparent in all cervical and thoracic centra.

Accordingly, where reference is made to performing a method at 5, 8 or 10 days, an alternative definition of the required time is by reference to the equivalent developmental stage, i.e. 5 days for zebrafish under standard conditions equates to appearance of the features listed at (i) above under whatever conditions the zebrafish are grown, which may for example be earlier that five days for zebrafish grown at temperature greater than 28.5 °C or later than five days for zebrafish grown at temperature lower than 28.5 °C . Similarly 8 days equates to appearance of the features listed at (ii) above and 10 days equates to appearance of the features listed at (iii) above.
Taking into account variation arising from alteration of conditions, molecules of less than 960 daltons may be excluded by between day 7 and 13, and especially between day 9 and 11, and in particular by day 10, with active efflux where applicable by between day 6 and 12, and especially between day 7 and 10, and in particular by day 8.

The present invention for example provides a method of screening for a substance with the desire effect wherein the method is substantially as set out in Figure 1. "Compound x" refers to a test substance.

The present invention provides a solution to technical problems associated with lack of knowledge in relation to the blood brain barrier.

In the absence of knowledge about formation of a blood brain barrier in zebrafish, false negative or false positive results may be obtained when searching for substances with an effect on the brain or central nervous system.

For example, if screening is performed after the blood brain barrier has formed (as determined by the present inventors) then a substance that could exert the desired biological effect if it were delivered to the brain but does not exert the desired biological effect when not delivered specifically to the brain because it does not cross the blood brain barrier will yield a false negative result. A person testing that substance without knowing that there is a blood brain barrier across which the substance cannot pass will discard the substance as not useful for achieving the desired biological effect, even though if the right delivery were used (e.g. directly into the brain or eye) the substance would in fact be useful for achieving that biological effect.

On the other hand, if such a search is performed using zebrafish prior to formation of the blood brain barrier (as determined by the present inventors), then a result may be obtained equivalent to a false positive result. For instance, a substance may exert the desired biological effect in the screen, but then later fail in adults because it is not able to cross the blood brain barrier. The substance may then be discarded as not useful, after some significant input of effort in the light of the initially positive result in the screen. Assessing ability to cross the blood brain barrier and, moreover, employing test fish at an appropriate stage of development, taking into account the knowledge provided by the present invention, avoids such problems and is of great benefit in the art.

In accordance with aspects and embodiments of the present invention, applying knowledge of the existence and time of formation of the blood brain barrier in zebrafish, the ordinary skilled person will obtain better and more useful results with test substances. For instance, a substance known not to be able to cross the blood brain barrier may be tested for desired biological activity in a screen that is designed taking into account the presence or absence of a blood brain barrier in the fish employed in the screen. Fish embryos may be used prior to their formation of a blood brain barrier and substances found to have the desired biological effect. Additionally or alternatively, substances may be tested in fish in which a blood brain barrier has formed, thereby establishing not only ability to exert the desired biological activity but also ability to cross the blood brain barrier. An option is to deliver the substance directly to the brain in a fish in which the blood brain barrier is known to have formed, thereby testing its ability to exert the desired biological effect without limitation as to its ability to cross the blood brain barrier.

In further embodiments, the invention provides for screening methods for substances that exert a desired biological activity in the body but also an undesired biological activity on a target that exists in the brain, central nervous system and/or eye. Thus, a screening method may be employed to determine biological activity of a test substance and to determine ability or inability of the test substance to cross the blood brain barrier, e.g. by determining whether or not the substance appears or appears to any significant extent within the brain, central nervous system or eye when not administered directly to these tissues. Knowledge about the existence of the bloods brain barrier in zebrafish and time of its establishment allows for screening methods to be designed in which exclusion of substances from the brain, central nervous system and/or the eye by virtue of inability to cross the blood brain barrier can be determined, and employed for example in selection of lead compounds for further development as drugs.

The skilled person is well versed in design and implementation of biological screens and application of appropriate control experiments. The present invention extends to screens in zebrafish that take into account the existence of the blood brain barrier in zebrafish and the time of its formation.

In various aspects and embodiments the present invention provides the subject-matter set out in the claims below.

It can additionally be determined whether a molecule is metabolised by the blood brain barrier by assessing the metabolite profile by HPLC for a test substance administered both before and after BBB formation.

The invention is generally applicable to any of a variety of diseases and disorders, and a range of examples is specifically set out as follows : The following diseases are common disorders of the central nervous system. A common feature of all these disorders is that the cell to be targeted by a therapeutic lies behind a cellular barrier - the blood brain, or blood retinal barrier.

Retinal degenerations, including:
Macular degeneration
Retinitis pigmentosa
Ganglion cell degeneration (glaucoma)

Demyelingating disorders, including:
Multiple sclerosis
ADEM

Degenerative disorders, including:
Parkinson's Disease
Alzheimer's Disease
Huntington's Disease
Diseases with motor neuron inclusions
Tauopathies
Corticobasal degeneration

Neuropsychiatric disorders, including:
Depression
Bipolar disease
Schizophrenia
Anxiety
Aggression
Sexual dysfunction

Miscellaneous, including:
Epilepsy
Headache
Pain
Sleep disorders
Satiety
CNS / retinal malignancies

Rarer conditions of the CNS and retina can be found in any standard medical textbook.

The present invention provides for formulation of an algorithm to apply in design of screening assays, taking into account the existence of the blood brain barrier in zebrafish (as determined by the present inventors) and the time of formation of the blood brain barrier, optionally also whether or not a test substance is known to be able or not to be able to penetrate the blood brain barrier. By means of an algorithmic approach, the skilled person determines which combination or combinations of experiments employing zebrafish of particular age, mode and site of delivery of test substance, nature of test substance, method of determining effect and so on are employed in the screening method. Furthermore, such an approach allows for interpretation of results obtained in different experiments, e.g. as illustrated in Figure 3.

The test zebrafish may have one or more symptoms or signs of a disease or disorder prior to use in screening for a substance with the desired activity.

The test substance may be administered with or prior to the administration of a substance which also induces one or more symptoms or signs of the disease or disease of interest.

Thus the invention is equally applicable to screening for - substances which exert a biological effect that alters an activity or function in the central nervous system, brain or eye, whether normal or subject to a disease or disorder, as to screening for substances which exert a biological effect that is ameliorative of a sign or symptom of a disease or disorder, is therapeutic or is prophylactic.

The test organism is a zebrafish.

The zebrafish is an organism which combines many of the advantages of mammalian and invertebrate model systems. It is a vertebrate and thus more relevant in models of human disease than *Drosophila* or other invertebrates, but unlike other vertebrate models it can be used to perform genetic screens.

A number of peer reviewed papers highlight and validate the use of zebrafish as a species in which to model human disorders. [Dooley K and Zon LI (2000) Zebrafish: a model system for the study of human disease. Current Opinion in Genetics and Development 10 : 252-6 -Barut BA and Zon LI (2000) Realising the potential of Zebrafish as a model for human disease Physiological Genomics 13: 49-51 - Fishman MC (2001) Zebrafish: The Canonical Vertebrate. Science 294: 1290-1].

The inventors have appreciated that zebrafish offer the unique combination of invertebrate scalability and vertebrate modelling capabilities. They develop rapidly, with the basic body plan already having been laid out within 24 hours of fertilization. Moreover, their ex-utero development within a transparent capsule allows the easy *in vivo* visualisation of internal organs through a dissecting microscope. Many disease states can be modelled within the first week of life, at which time the embryos are only a few millimetres long and capable of living in 100 µl of fluid. This permits analysis of individual embryos in multi-channel format, such as 96 well plate format. This is particularly useful for drug screening, with many chemicals being arranged in 96 well plate format.

A population of zebrafish in a petri dish or a tank may be employed. A population of fish may be treated together, and may be tested together, e.g. via addition of one or more or a combination of test substances to the water.

The zebrafish has a short maturation period of two to three months and is highly fecund, with a single pair of adults capable of producing 100 to 200 offspring per week. Both embryos and adults are small, embryos being a few mm and adults 2-3 cm long. They are cheap and easy to maintain. The ability to generate large numbers of offspring in a small place offers the potential of large scalability.

Zebrafish are thus a valid organism for screening for substances with biological activity in vertebrates, including mammals, including humans, which substances are useful in vivo, for example as therapeutics. The present invention extends the value of zebrafish screens by allowing for improved design of screens and improved usefulness of results obtained.

A further advantage of zebrafish is the fact they live in water. This makes administration of test substances easy as they may be added to water in which the fish are. Zebrafish readily absorb chemicals. The effective concentration of chemicals in the water often equates to the effective plasma concentration in mammals.

Different test substances may be added to each well of a multi-well plate, such as a 96 well plate, to identify that test substance exhibiting a beneficial or deleterious effect. There may be one or multiple fish in each well exposed to the test substance.

Zebrafish are also DMSO (dimethyl sulphoxide) tolerant. This is important as DMSO is used as a solvent to dissolve many drugs. The inventors have established that zebrafish can tolerate 1% DMSO. Thus, a candidate drug or other test substance may be dissolved in DMSO and administered to zebrafish by adding to the fish water to give a final concentration of DMSO of at least up to 1%. This is employed in various preferred aspects and embodiments of the present invention.

The same test substance may be added to different wells at a different concentration. For example, test substance 1 may be added to well A1 at a concentration of 1mM, to well A2 at a concentration of 1OOuM, to well A3 at a concentration of 10uM, to well A4 at a concentration of 1uM and to well A5 at a concentration of 0.1uM. Then test substance 2 to well B1 etc. The panel of test substances may be known drugs or new chemical entities.

Additionally, the test substances may be added in combination. For example, well A2 may contain test substance 1 and 2, well A3 test substance 1 and 3, well B2 test substance 2 and 3. Alternatively, every well may contain test substance x, with individual wells containing a panel of additional test substances.

In other options, a population of zebrafish in a petri dish or a tank may be employed and treated together, e.g. via addition of one or more or a combination of test substances in the water.

For delivery into the brain a delivery system may be employed, for example using a lipophilic delivery molecule, with the whole "trojan horse" being administered to the fish water. Alternatively, the test substance may be injected directly with the aid of a micromanipulator, into the CNS, for example into a ventricle, thus bypassing the BBB.

Thus, zebrafish enable the entire biological pathway of a vertebrate to be screened in a high-throughput fashion.

The present invention in certain aspects and embodiments provides for screening for and preferably identifying or obtaining a substance that provides a synergistic combination with another substance, or for screening for and preferably identifying or obtaining two or more substances that together provide an additive or synergistic combination. Clinical benefit is often derived from synergistic combinations of drugs. Use of a screening system in accordance with the present invention allows for identification of such synergistic combinations.

Thus, in certain embodiments the invention comprises treating the zebrafish, as discussed, with two or more substances, at least one of which is a test substance, and comparing the effect of the two or more substances in combination to determine the optimum effect (whether simultaneously or sequentially applied) on an aspect of behaviour or physiology with the effect of either or both of the two or more substances when applied individually or alone. Either all (or both) of the substances applied may each be a test substance, or one of the substances may be a drug known to have a beneficial effect in the disease that is the subject of the screen, or at least an effect in the test fish.

The invention thus provides for screening for and preferably identifying or obtaining a substance that provides an additive effect to a known drug or a synergistic effect with the known drug. It also provides for screening for and preferably identifying or obtaining a combination of two or more substances that provide a synergistic effect, compared with the effect of the two substances when employed individually or alone.

Add-on therapies are useful because it is difficult to conduct clinical trials in which an existing drug is withdrawn from a patient and replaced with a new drug. The patient is deprived of a drug which has at least got some proven efficacy and some confidence in its side-effect profile. Additionally, the patient will be vulnerable to their disease during the phases of withdrawal of the existing drug and build up of the test drug.

The test zebrafish may be mutated rather than a wild-type. It is then possible to assay for interacting effects, either beneficial synergistic effects, or deleterious effects, of the mutation plus the test substances. Alternatively, the analysis may be of the known therapeutic agent and the genetic mutation to discover either a new drug target of benefit in combination with the known drug, or a genetic marker of use in predicting which patients are most likely to benefit (or not benefit) from prescription of the known drug.

In another embodiment, a combination of potentially useful agents is administered to a test zebrafish having one or more symptoms of a disease or disorder, which may be generated through addition of an agent to the test fish, e.g. via addition to the fish water or through expression or knock out of a gene, to assess whether the combination is more effective than either of the individual agents.

The present invention also provides for screening for and preferably identifying or obtaining a substance that ameliorates one or more side effects of an active substance, e.g. a therapeutically active substance. There are many drugs which have been discontinued in clinical trials, or are marketed but infrequently prescribed, not because they are not therapeutically effective, but because their side-effect profile is limiting. The side-effects may be relatively benign, but significant to the patient, such as renal damage (e.g. cyclosporin). It is desirable to allow the administration of such drugs, with proven beneficial effects, through the co-administration of an additional agent to improve the side-effect profile.

In accordance with the present invention, such agents are screened for in zebrafish in which administration of the active substance induces a side-effect or other phenotype reflective or indicative of a side-effect. Thus in embodiments of the invention, an active agent is administered to test zebrafish having one or more symptoms of an autoimmune disease and the side-effect of other phenotype is assessed for such fish when subjected to one or more test substances. This does not require *a priori* knowledge of action of the co-administered agent. In other embodiments, agents that achieve the desired therapeutic effect with a reduction of side-effects can be screened for and preferably identified or obtained by means of assessment of disease phenotype and side-effect phenotype. As with other aspects and embodiments of the present invention, this may involve co-administration of a primary compound together with either a battery of candidate substances, or together with randomly induced genetic mutation. With the latter approach, i.e. mutation, subsequent steps are needed to identify the appropriate co-therapeutic following identification of fish with a mutation that provides an ameliorative effect.

A diverse library of drug-like compounds, such as the LOPAC library (Sigma) may be used, or the Chembridge PHARMACOphore diverse combinatorial library. Other targeted libraries against particular targets classes may be used, such as ion channel libraries or G protein libraries.

Following identification of an active substance using a screen in accordance with the present invention, the substance may be used in a method of medical treatment of the present invention, with administration preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors.
1. A test substance is added to the test fish either prior to the appearance of the disease state, at the time of induction of the disease state, or after the induction of the disease state. The first two situations are more likely to identify a prophylactic chemical, the latter a drug which reverts the disease state back to normal. The test substance may be a chemical and may be a random chemical administered in a high-throughput fashion to fish in 96 well plate format, or a selected chemical administered to a clutch of fish in a Petri dish.
2. The fish is then screened for deviation from the initial disease state.

The following additional steps are highly desirable in screening, and their use is provided by the present invention in preferred embodiments:
Rather than add a single chemical, a combination of chemicals is added. For instance, a known therapeutic agent may be administered to all fish at a dose at which a further beneficial effect could still be detected. A random chemical library is then added to fish and an incremental effect screened for.
   1. Induce an autoimmune disease state by the administration of antibodies to fish.
   2. Administer drug 1 to row 1, drug 2 to row 2, etc.
   3. Administer drug 1 to column 1, drug 2 to column 2 etc.
   4. Compare the immunosuppressive effect of all wells with that of the drugs when given alone.
A further embodiment of the above allows for detection of augmentation of a particular drug through a particular mutation, as follows:
   1. Induce genetic mutation through any of the above.
   2. Induce disease state through any of the above.
   3. Administer test chemical.
   4. Assess whether the combination of the mutation plus chemical is greater than either alone.
   5. The mutated gene is then used as a beneficial target, as described above.

A further embodiment of the invention allows identification of genetic factors which help determine the appropriateness of a particular therapeutic agent for a given patient. If the mutation augments the effect of the drug, that mutation is searched for in human homologues. Patients with this mutation should be preferentially prescribed the drug. If the mutation leads to a deleterious effect or lack of effect, then patients should avoid this drug.

### EXPERIMENTAL

Evans Blue dye (961 Da) was used to investigate the presence of BBB in zebrafish larvae. Evans Blue is used in rodent studies of BBB and is excluded from the brain if the BBB is intact. In rodents, Evans Blue leakage is observed following brain trauma.
1) 2% Evans Blue in 0.9% saline was injected into pericardial sac of larvae from 2 days post-fertilisation (d.p.f.) to 1 month old. 0.9% saline was injected as a control.
2) The larvae were left to develop for 6 hours to allow the dye to penetrate from bloodstream into tissues.
3) The larvae were viewed under fluorescent dissecting scope (TRITC filter) to ensure that dye was present in blood vessels.
4) Larvae were anaesthetised and fixed in 4% PFA at 4 °C overnight.
5) They were embedded in O.C.T. (Optimal Cutting Temperature compound) and 10 µm frozen sections were taken in parasaggital plane.
6) Midline sections were viewed on fluorescent microscope (TRITC filter) at x40 magnification. Images were taken of Evans Blue injected and saline injected samples.
7) Fluorescent intensity was measured in 3 regions of control and Evans Blue injected samples using Analysis software. Mean fluorescent intensity was calculated for each age group.
8) Mean fluorescent intensity in Evans Blue injected vs saline controls was compared by means of a histogram (Figure 2).

### Resul ts

High level of fluorescence in the brain at 3 d.p.f. provided indication that Evans Blue crossed into the brain and the BBB was not established. By 5 d.p.f., brain fluorescence in Evans Blue injected samples was no different from saline injected samples, demonstrating that Evans Blue was excluded and providing indication that the BBB had developed by 5 d.p.f.

### METHODS

### Husbandry

Embryos were collected from matings of adults (TL, AB and WIK strains), reared under standard conditions (Westerfield, 1995). Embryos were reared in embryo medium (5 mM NaCl, 0.17 mM KCl,0.33 mMCaCl₂, 0.33 mM Mg₂SO₄, 10⁻⁵% Methylene Blue) and staged using standard criteria (Kimmel et al., 1995).

### Dye exclusion experiments

Injection stocks of 2% evans blue in 0.9% saline and a 10% sodium fluorescein in 0.9% saline were prepared and stored at 4 °C. Larvae were anaesthetised by immersion in 0.2 mg/ml 3-amino benzoic acid (MS222) prior to being immobilised and oriented in 3% methyl cellulose in embryo medium in a lateral position such that the heart was accessible. Larvae and juveniles from 2 days post-fertilisation (d.p.f.) to 30 d.p.f. were injected into the pericardial region with 0.5 to 2 nl of dye or saline control, depending on age, using standard zebrafish egg microinjection apparatus (Westerfield, 1995). Fish were transferred to fresh embryo medium to recover from anaesthesia, then viewed using a fluorescence dissecting microscope at 1 hour intervals to determine the time of uptake of the dye into the circulation and the time at which dye had permeated from the circulation into non-vascular body tissue. Dye and saline injected larvae were anaesthetised at 4 hours and 6 hours after injection and fixed in 4% paraformaldehyde in PBS. 10 larvae per age group and injection treatment were embedded in Sakura Tissue-Tek OCT Compound (Bayer, Newbury, UK) and frozen parasagittal sections were cut at 10 µm thickness.

### Quantification of dye fluorescence

Sections were visualised without mounting medium by fluorescence microscopy on an Olympus BX51 microscope. Three midline sagittal sections from each larva were identified and images were captured using a ColorView camera (Olympus) and AnalySis software (Soft Imaging System). The fluorescence intensity within the brain was quantified for each midline section of each larva in each treatment group using colour threshold and area measurements in AnalySis. Mean values, standard deviations and T-tests were calculated using Excel software (Microsoft Office).

### Transmission Electron Microscopy

Larvae at 3, 5, 8 and 10 d.p.f. were fixed in 4% glutaraldehyde in cacodylate buffer containing 0.006% hydrogen peroxide for 3 hours, then washed in cacodylate buffer before post-fixing in osmium tetroxide. Samples were bulk stained with uranyl acetate, dehydrated in ethanol and embedded in Spurr's resin. Thin sections (5 nm) were prepared with a Leica Ultracut UCT, stained with uranyl acetate and lead citrate and viewed in a Philips CM100 electron microscope at 80 KV.

### Pgp expression

Larvae, at 3, 5, 8 and 10 d.p.f., were fixed in 4% PFA and processed for cryosectioning as described above. 10 µm sections were then washed in PBS and Pgp (Abcam) antibody staining was performed at 1:100 dilution and the staining detected using Alexa 568 fluorescent secondary antibody (Molecular probes). Sections were counterstained and mounted using Vectamount containing DAPI (Vector Laboratories, Peterborough, UK) then viewed using an Olympus BX51 microscope under fluorescence illumination. Representative images were captured using a ColorView camera (Olympus) and AnalySis software (Soft Imaging System).

### Drug exposure and distribution experiments

Approximately 100 larvae were tranferred into a single well of a 12 well plate (Corning) containing embryo medium. The final volume of embryo medium per well was adjusted to 1.5 ml. Compounds of interest were prepared as 2 mg/ml frozen stocks in DMSO. 11.25 µl of stock was added to each well to give a final concentration of 15 µg/ml. Larvae were incubated in the drug for 1 hour at room temperature, then anaesthetised on ice. For whole uptake analysis, anaesthetised larvae were transferred to small microfuge tubes and all excess liquid removed. For brain versus body uptake analysis, larvae were anaesthised by chilling then decapitated using fine iridectomy scissors. Head and body tissue samples were collected in separate microfuge tubes on ice and all excess liquid removed. The total weight of tissue sample was measured. The samples were then frozen at -20 °C prior to extraction for HPLC. For subsequent HPLC analysis, tissue samples were thawed and an initial extraction performed using tertiary-butyl methyl ether (t-bme). The solvent extract was then evaporated and reconstituted in 1M acetate buffer pH 4.7. Further clean-up was obtained using a mixed-mode (cation-exchange) solid-phase extraction method. The eluate was evaporated and reconstituted in methanol/acetic acid. 10 ml injections of extracted samples were made onto a LCMS system using a Waters SymmetryShield (150 x 3.9) RP8 analytical column. Samples were analysed by full scan LCMS/MS under APCI conditions in positive ion mode. LC conditions are given in Table 1. MS/MS scan parameters are given in Table 2.

Additional modifications were required for the extraction of simvastatin and pravastatin, in that 10 µL injections were made onto the LCMS system using a Luna C18 (5 x 4.6) analytical column. Samples were analysed by full scan LCMS/MS under ESI conditions in positive ion mode for simvastatin and negative ion mode for pravastatin. LC conditions are given in Table 3. MS scan parameters are given in Table 4.

### The effect of Pgp inhibition on the distribution of rhodamine 123

Rhodamine 123 was dissolved in 10% EtOH in 0.9% saline to make a 0.5 mg/ml stock. Dye exclusion experiments on larvae at 3, 5, 8 and 10 d.p.f. were performed as described above using 0.5 mg/ml rhodamine 123 in the presence or absence of verapamil. Larvae were exposed to verapamil at 50 µg/ml for 2 hours prior to and during intrapericardial injection of the dye and for 3 hours after injection. At 3 hours after injection, larvae were anaesthetised and fixed in 4% PFA prior to processing for frozen sections. Quantification of fluorescence in the brain was performed as described above.

### Behavioural analysis following drug exposure

Larvae at 3, 5 and 10 d.p.f. were transferred into 96 well plates (Millipore) containing embryo medium. Behavioural analysis was performed in 96 well plates with 1 fish per well. The final volume of embryo medium per well was adjusted to 200 µl. Master stocks of test compounds were made at 100x test concentration. Test compounds were anti-histamine drugs selected on the basis of their ability to cross the BBB in mammals; diphenhydramine, a sedating drug which does cross the BBB; desloratidine, a non-sedating drug that does not cross the BBB. 2 µl of master stock of test compound was added to each well of the screening plate. 12 wells were set up for each compound at each larval age. Activity was recorded within individual wells of the 96 well plate over an 2 hour period using Ethovision TrackSys equipment and software. Mean total activity was calculated for each treatment group at each larval age using Excel software.

### RESULTS

### Zebrafish show anatomical evidence of a blood brain barrier

TEM at 3 d.p.f shows no evidence of tight junctions. In contrast TEM at 5 d.p.f. shows tight junctions are present in some but not all vascular endothelium, with a gradual maturation henceforth.

### Zebrafish possess a pgp orthologue

In silico cloning identified a potential zebrafish orthologue of human Pgp.

### Zebrafish express PGP in a developmentally regulated fashion

Pgp is expressed in the vascular endothelium of the CNS at 8 d.p.f and stages thereafter..

### The formation of a blood brain barrier correlates developmental changes in drug distribution

Drugs that do not cross the BBB are present throughout the body of samples at 3 d.p.f. but are excluded from the head at 10 d.p.f. Drugs that cross the BBB are equally distributed in samples at 3 d.p.f. and at 10 d.p.f.

The fluorescence intensity of test compound and control agent was measured in the brain of zebrafish following peripheral injection at various time points. Sodium fluorescein, like all other small molecules tested, permeates into the brain until day 8 but is excluded from day 10.

In contrast, Evan's blue, a much larger molecule known to form multimers, is excluded from the brain from day 5.

### The presence of a blood brain barrier correlates with the functional effects of drugs

Antihistamines known to be non-sedating in humans due to their exclusion from the brain were observed not to have a sedating effect on d10 zebrafish, in contrast to those antihistamines known to have a sedating effect in humans on account of their penetration into the brain, which also had a sedating effect on d10 zebrafish.

### Zebrafish show active transport of compounds across their blood brain barrier

R123 is rhodamine123, a fluorescent substrate for pgp. Co-administration of verapamil, an inhibitor of pgp, leads to penetration of rhodamine 123 into the brain at time points when it would otherwise be excluded, demonstrating active efflux of rhodamine 123.

### DISCUSSION

### Zebrafish have a developmentally regulated blood brain barrier (BBB)

The creation of a selective compartment through the formation of an epithelial barrier, utilises two types of junctions: tight junctions and septate junctions. Both junctions are selective and dynamic and can link to intracellular signal and pathways through membrane associated proteins.

Fish brain epithelial barriers have been studied anatomically in a number of species by electron microscopy (Nakao, 1979). On ultrathin sectional electron microscopy, tight junctions appear at the zone of complete contact between the plasma membranes of adjacent cells (Farquhar and Palade, 1965). On freeze fracture electromicroscopy, they appear as a continuous anastomosing network of tight junction strands of ripples and complementary grooves (Staehelin, 1974). Rascher and Wolburg (Rascher and Wolburg, 1997) looked at ultrathin sections of adult carp and showed tight, and gap junctions and desmosomes in the arachnoid membrane. The same paper demonstrated a gradual maturation of the tight junctions in chick. They conclude their discussion with the statement (last sentence, last page): the large variability pf the anatomy of the meningeal layers in vertebrates and - as we have shown here - of the tight junction structure make it more difficult to elucidate the mechanism of arachnoid barrier induction and to transfer a result from one species to another

Through the peripheral administration of sodium fluorescein, of molecular weight 376 Da, we have been able to show that zebrafish do indeed have a blood brain barrier. This begins to form on day 5, with the result.that larger molecules such as Evan's blue (which may sometimes form multimers), are excluded from this time. The BBB remains leaky, however, to small molecules, such as drug-like small molecules until the BBB has further matured on day 10, unless there is active efflux by pgp, in which case effective exclusion occurs from day 8.

### The zebrafish BBB possesses functional active transport systems

A good clinical example of the role of p-glycoprotein efflux is the explanation for the non-sedating effects of second-generation antihistamines, these being actively effluxed from the brain unlike their first generation counterparts (Chen et al., 2003). For example, hydroxyzine, diphenhydramine and triprolidine are all sedating antihistamines, whereas loratadine, desloratadine (the active metabolite of loratadine), and cetirizine (the active metabolite of hydroxyzine) are non-sedating.

Pgp is present in epithelial cells specialised in the secretion and excretion of undesired molecules, particularly in the gut, liver, kidney and BBB. P-glycoproteins undergo extensive post-translational modifications, including phosphorylation and glycosylation. There are at least 3 types of p-glycoprotein type in mammals. Bard (aquatic toxicology 2000) summarise expression of pgp-like proteins in fish in figure 2, although do not report BBB expression.

We have shown here both anatomical and functional evidence for active transport of molecules across the blood brain barrier in zebrafish. Thus whilst non-pgp substrate small molecules can penetrate the BBB until day 10, those which are pgp substrates are actively effluxed from day 8.

### A zebrafish model for BBB compound penetration

Attempts to predict drug permeation through membranes includes measurements of drug partitioning in solvents, although perfusion through solvents is not identical to diffusion across biological membranes, never mind the lack of information about active transport systems (Lieb and Stein, 1976). Indeed, as a result of transport proteins such as the p-glycoproteins the net effects of a variety of hydrophobic drugs, such as digoxin, cyclosporin and loperamide, is relatively low (Schinkel et al., 1996).

Models of blood-brain barrier involving cell culture systems play some role in assessing whether drugs may cross the blood-brain barrier, but are limited in that transport systems and blood-brain barrier enzymes may be severely down regulated or not present at all (Terasaki et al., 2003). Furthermore, they do not form rigid tight junctions.

Zebrafish may therefore be employed very usefully in determining whether a compound penetrated the BBB in an in vivo situation. Moreover, these results allow for the rational screening of small molecules for CNS and ophthalmological endpoints in zebrafish.

**TABLE 1**

| *LC conditions* | | | |
|---|---|---|---|
| **Time (minutes)** | **% acetic acid (0.1%)** | **% acetonitrile** | **Flow Rate mLmin⁻¹** |
| 0.0 | 95 | 5 | 1.0 |
| 3.5 | 50 | 50 | 1.0 |
| 5.0 | 30 | 70 | 1.0 |
| 6.0 | 0 | 100 | 1.0 |
| 6.5 | 95 | 5 | 1.0 |

**TABLE 2**

| *MS Scan Parameters* | | | |
|---|---|---|---|
| **Drug** | **[M+H]⁺** | **CE** | **Isolation Width** |
| Loperamide | 479.0 | 39 | 4.0 |
| Haloperidol | 377.0 | 50 | 4.0 |
| Scopolamine | 304.1 | 36 | 2.0 |
| Diphenhydramine | 255.9 | 28 | 2.0 |

**TABLE 3**

| *LC conditions for pravastatin and simvastatin* | | | | | |
|---|---|---|---|---|---|
| **Time (minutes)** | **% 1mM acetate buffer** | | **% acetonitrile** | | **Flow Rate mLmin⁻¹** |
| | **Sim** | **Prav** | **Sim** | **Prav** | |
| 0.0 | 40 | 60 | 60 | 40 | 0.3 |
| 3.0 | 20 | 20 | 80 | 80 | 0.3 |
| 3.1 | 0 | 0 | 100 | 100 | 0.3 |
| 4.0 | 0 | 0 | 100 | 100 | 0.3 |
| 4.1 | 40 | 60 | 60 | 40 | 0.3 |
| 5.0 | 40 | 60 | 60 | 40 | 0.3 |

**TABLE 4**

| *MS Scan Parameters for pravastatin and simvastatin* | | | |
|---|---|---|---|
| **Drug** | **[M+H]⁺** | **CE** | **Isolation Width** |
| Simvastatin | 419.1 | 22.0 | 3.0 |
| Pravastatin | 423.1 | 30.0 | 2.1 |

### REFERENCES

Aldegunde *et al***.** J Exp Zool 2000; 286: 131-5.
Anichtchik *et al.* J Neurochem 2004; 88: 443-53.
Barut, B.A. and Zon, L.I. (2000). Physiological Genomics 13: 49-51.
Borg-Neczak *et al.* Toxicol Lett 1996; 84: 107-12.
Brightman Exp Eye Res 1977; 25 Suppl: 1-25.
Chen *et al.* Drug Metab Dispos 2003; 31: 312-8.
Dohrmann GJ. The choroid plexus: a historical review. Brain Res 1970; 18: 197-218.
Dooley, K. and Zon, L.I. (2000). Current Opinion in Genetics and Development 10: 252-256.
Farquhar *et al.* J Cell Biol 1965; 26: 263-91.
Fishman, M.C. (2001). Science 294: 1290-1291.
Goldsmith P. Curr Opin Pharmacol 2004; 4: 504-12.
Goldsmith *et al.* J Neurobiol 2003; 57: 235-45.
Ibiwoye *et al.* (1994) J Comp Pathol. 111: 43-53.
Khan, N. and Deschaux, P. (1997). J Exp Biol. 200: 1833-1838.
Kimmel *et al.* Dev Dyn 1995; 203: 253-310.
Langheinrich Bioessays 2003; 25: 904-12.
Lieb *et al*. Biochim Biophys Acta 1976; 455: 913-27.
Nag, S. (2003)Methods Mol Med. 89: 3-36.
Nakao J Comp Neurol 1979; 183: 429-53.
Nitta *et al.* J Cell Biol 2003; 161: 653-60.
Pardridge Neuron 2002; 36: 555-8.
Pardridge Methods Mol Med 2003; 89: 385-99.
Rascher J Hirnforsch 1997; 38: 525-40.
Schinkel *et al.* Clin Invest 1996; 97: 2517-24.
Schumacher *et al.* Histochem Cell Biol 1997; 108: 179-82.
Staehelin Int Rev Cytol 1974; 39: 191-283.
Stewart *et al.* Brain Res 1987; 429: 271-81.
Terasaki *et al.* Drug Discov Today 2003; 8: 944-54.
Tsuji *et al.* Adv Drug Deliv Rev 1999; 36: 277-290.
Westerfield M 1995 The Zebrafish Book: A Guide for the Laboratory Use of Zebrafish (Danio Rerio*). University of Oregon Press, Eugene, Oregon.
Westerfield *et al*. Seminars in Cell and Developmental Biology 1997; 8: 477-88.
Whitfield J Neurobiol 2002; 53: 157-71. Wolburg *et al*. Vascul Pharmacol 2002; 38: 323-37.

## Claims

1. A screening method to obtain a substance with biological activity in the central nervous system (CNS), brain or eye, the method comprising:
administering a test substance to test zebrafish
determining effect of the test substance on an activity or function of the central nervous system, brain or eye, or effect of the test substance on a symptom or a disease or disorder of the central nervous system, brain or eye of the test fish,
thereby identifying a substance with said biological activity;
wherein:
the test substance is known to penetrate the blood brain barrier and the method is performed on zebrafish of any age;
or the test substance is known not to penetrate the blood brain barrier and the method is performed on zebrafish of age less than five days post-fertilisation for molecules of 960 daltons or greater, less than ten days post-fertilization for molecules of less than 960 daltons not effluxed by p-glycoprotein (pgp) or less than eight days post-fertilization for molecules actively effluxed by pgp, and/or is performed on zebrafish of age at least five days post-fertilisation for molecules of 960 daltons or greater, or at least ten days post-fertilization for molecules of less than 960 daltons not effluxed by pgp and at least eight days post-fertilization for molecules actively effluxed by pgp, and comprises direct delivery of the test substance to bypass the blood brain barrier;
or, without knowing whether or not the test substance is able to penetrate the blood brain barrier, the method is performed on zebrafish of age at least five days post-fertilisation for molecules of 960 daltons or greater, and at least ten days post-fertilization for molecules of less than 960 daltons, with direct delivery of the test substance to bypass the blood brain barrier;
where the stated ages refer to zebrafish grown under standard conditions and are adjusted if the zebrafish are grown under conditions that accelerate or retard achievement of the equivalent stage of development.

2. A method according to claim 1 wherein said direct delivery comprises injection into the brain or eye.

3. A screening method to obtain a substance with biological activity in the central nervous system (CNS), brain or eye, the method comprising:
administering a test substance to test zebrafish fish,
determining effect of the test substance on an activity or function of the central nervous system, brain or eye, or effect of the test substance on a symptom or a disease or disorder of the central nervous system, brain or eye of the test fish,
thereby identifying a substance with said biological activity;
wherein the method is performed for molecules of 960 daltons or greater on zebrafish of age less than five days post-fertilisation and on zebrafish of age at least five days post-fertilisation , or is performed for molecules of less than 960 daltons on zebrafish of age less than ten days post-fertilisation and on zebrafish of age at least ten days post-fertilization, without direct delivery of the test substance to bypass the blood brain barrier, whereby a substance which has said biological activity and has ability to penetrate the blood brain barrier is obtained;
where the stated ages refer to zebrafish grown under standard conditions and are adjusted if the zebrafish are grown under conditions that accelerate or retard achievement of the equivalent stage of development.

4. A method for obtaining a substance with biological activity in the central nervous system (CNS), brain or eye, the method comprising a screening assay conducted by administering a test substance to test zebrafish whereby a substance with said biological activity is obtained, wherein design of the screening assay employs an algorithm formulated to take into account the presence of and time of formation of a blood brain barrier in the zebrafish.

5. A method according to claim 4 wherein the algorithm is further formulated to take into account whether or not the test substance crosses the blood brain barrier.

6. A screening method for a substance with biological activity in the central nervous system (CNS), brain or eye substantially as set out in Figure 1.

7. Use of the existence of and time of formation of a blood brain barrier in a zebrafish in the.design of a screening assay for a substance with biological activity in the central nervous system (CNS), brain or eye.

8. A screening method to obtain a substance with biological activity, which substance does not pass the blood brain barrier, the method comprising:
administering a test substance to test zebrafish
determining effect of the test substance on an activity or function in the zebrafish or effect of the test substance on a symptom or a disease or disorder in the test fish,
thereby identifying a substance with said biological activity,
wherein the method further comprises determining ability or inability of the substance with said biological activity to cross the blood brain barrier in test zebrafish of age at least five days post-fertilisation for molecules of 960 daltons or greater, or at least ten days post-fertilization for molecules of less than 960 daltons, thereby identifying a substance with said biological activity and which does not cross the blood brain barrier;
where the stated ages refer to zebrafish grown under standard conditions and are adjusted if the zebrafish are grown under conditions that accelerate or retard achievement of the equivalent stage of development.

9. A method according to claim 8 comprising identifying the substance with said biological activity in a test zebrafish of age less than five days post-fertilisation for a molecule of 960 daltons or greater, less than ten days post-fertilization for a molecule of less than 960 daltons not effluxed by pgp or less than eight days post-fertilization for a molecule actively effluxed by pgp; where the stated ages refer to zebrafish grown under standard conditions and are adjusted if the zebrafish are grown under conditions that accelerate or retard achievement of the equivalent stage of development.

10. use of the existence of and time of formation of a blood brain barrier in a zebrafish in the design of a screening assay for a substance with biological activity and which does not cross the blood brain barrier.

11. A method or use according to any one of the preceding claims comprising determining whether a test substance is actively transported across the blood brain barrier.

12. A method or use according to claim 11 comprising determining whether a test substance is actively transported into the brain

13. A method or use according to claim 11 comprising determining whether a test substance is actively transported out of the brain

14. A method or use according to any one of the preceding claims comprising determining whether a test substance is transported by p-glycoprotein.

15. A method or use according to any one of the preceding claims comprising determining whether a test substance interferes with p-glycoprotein function.

16. A method or use according to any one of the preceding claims comprising determining whether a test substance alters p-glycoprotein expression.

17. A method or use according to any one of claims 14 to 16 wherein the test substance is assessed both before and after development of functional p-glycoproteins.

18. A method or use according to claim 17 wherein the test substance is assessed on day 8 or 9 post-fertilisation as well as day 10 or after, where the stated ages refer to zebrafish grown under standard conditions and are adjusted if the zebrafish are grown under conditions that accelerate or retard achievement of the equivalent stage of development.

19. A method or use according to any one of the preceding claims comprising determination whether a combination of test substances have altered BBB penetration in comparison to a test substance given in isolation.

20. A method or use according to any one of the preceding claims comprising determination of body: brain drug concentration gradients.

21. A method or use according to any one of the preceding claims comprising comparing relative efficacies of test substances with desired activity in the central nervous system in the presence of a blood brain barrier.

22. A method or use according to any one of the preceding claims comprising determining penetration of a test substance across the blood-retinal barrier.

23. A method or use according to any one of the preceding claims comprising determining whether a test substance is metabolised by the blood brain barrier.

24. Use of zebrafish to determine whether a test substance is actively transported across the blood brain barrier.

25. Use of zebrafish to determine whether a test substance is actively transported into the brain.

26. Use of zebrafish to determine whether a test substance is actively transported out of the brain.

27. Use of zebrafish to determine whether a test substance is transported across the blood brain barrier by p-glycoprotein.

28. Use of zebrafish to determine whether a test substance interferes with p-glycoprotein function.

29. Use of zebrafish to determine whether a test substance alters p-glycoprotein expression.

30. Use according to any one of claims 27 to 29 wherein the test substance is assessed both before and after development of functional p-glycoproteins.

31. Use according to claim 30 wherein the test substance is assessed on day 8 or 9 post-fertilisation as well as day 10 or after, where the stated ages refer to zebrafish grown under standard conditions and are adjusted if the zebrafish are grown under conditions that accelerate or retard achievement of the equivalent stage of development.

32. Use of zebrafish to determine whether a combination of test substances have altered BBB penetration in comparison to a test substance given in isolation.

33. Use of zebrafish to determine body: brain drug concentration gradients.

34. Use of zebrafish to compare relative efficacies of test substances with desired CNS activity in the presence of a blood brain barrier.

35. Use of zebrafish to determine penetration of a test substance across the blood-retinal barrier.

36. Use of zebrafish to determine whether a test substance is metabolised by the blood brain barrier.

## Patentansprüche

1. Screening-Verfahren zum Erhalt einer Substanz mit biologischer Aktivität im Zentralnervensystem (ZNS), Hirn oder Auge, wobei das Verfahren Folgendes umfasst:
das Verabreichen einer Testsubstanz an einen Testzebrafisch,
das Bestimmen der Wirkung der Testsubstanz auf eine Aktivität oder Funktion des Zentralnervensystems, Hirns oder Auges oder der Wirkung der Testsubstanz auf ein Symptom oder eine Erkrankung oder Störung des Zentralnervensystems, Hirns oder Auges des Testfischs,
wodurch eine Substanz mit dieser biologischen Aktivität identifiziert wird;
worin:
die Testsubstanz die Blut-Hirn-Schranke bekannterweise überwindet und das Verfahren an einem Zebrafisch beliebigen Alters durchgeführt wird;
oder die Testsubstanz die Blut-Hirn-Schranke bekannterweise nicht überwindet und das Verfahren an einem Zebrafrisch mit einem Alter von weniger als fünf Tagen nach der Befruchtung für Moleküle mit 960 Dalton oder mehr, weniger als 10 Tagen nach der Befruchtung für Moleküle mit weniger als 960 Dalton, die nicht durch ein p-Glykoprotein (pgp) ausgeschleust werden, oder weniger als 8 Tagen nach der Befruchtung für Moleküle, die aktiv durch gpg ausgeschleust werden, durchgeführt wird und/oder an einem Zebrafisch mit einem Alter von zumindest 5 Tagen nach der Befruchtung für Moleküle mit 960 Dalton oder mehr oder zumindest 10 Tagen nach der Befruchtung für Moleküle mit weniger als 960 Dalton, die nicht durch pgp ausgeschleust werden, und zumindest 8 Tagen nach der Befruchtung für Moleküle, die aktiv durch pgp ausgeschleust werden, durchgeführt wird und die direkte Zufuhr der Testsubstanz umfasst, um die Blut-Hirn-Schranke zu umgehen;
oder das Verfahren, ohne zu wissen, ob die Testsubstanz die Blut-Hirn-Schranke überwinden kann, an einem Zebrafisch mit einem Alter von zumindest 5 Tagen nach der Befruchtung für Moleküle mit 960 Dalton oder mehr und zumindest 10 Tagen nach der Befruchtung für Moleküle mit weniger als 960 Dalton durchgeführt wird, wobei die Testsubstanz direkt zugeführt wird, um die Blut-Hirn-Schranke zu umgehen;
worin sich die angegebenen Alterswerte auf Zebrafische beziehen, die unter Standardbedingungen gezüchtet wurden, und angepasst werden, wenn ein Zebrafisch unter Bedingungen gezüchtet wurde, die das Erreichen eines äquivalenten Entwicklungsstadiums beschleunigen oder verzögern.

2. Verfahren nach Anspruch 1, worin die direkte Zufuhr eine Injektion in das Hirn oder ein Auge umfasst.

3. Screening-Verfahren zum Erhalt einer Substanz mit biologischer Aktivität im Zentralnervensystem (ZNS), Hirn oder Auge, wobei das Verfahren Folgendes umfasst:
das Verabreichen einer Testsubstanz an einen Testzebrafisch,
das Bestimmen der Wirkung der Testsubstanz auf eine Aktivität oder Funktion des Zentralnervensystems, Hirns oder Auges oder der Wirkung der Testsubstanz auf ein Symptom oder eine Erkrankung oder Störung des Zentralnervensystems, Hirns oder Auges des Testfischs,
wodurch eine Substanz mit dieser biologischen Aktivität identifiziert wird;
worin:
das Verfahren für Moleküle mit 960 Dalton oder mehr an einem Zebrafisch mit einem Alter von weniger als 5 Tagen nach der Befruchtung und an einem Zebrafisch mit einem Alter von zumindest 5 Tagen nach der Befruchtung durchgeführt wird oder für Moleküle mit weniger als 960 Dalton an einem Zebrafisch mit einem Alter von weniger als 10 Tagen nach der Befruchtung und an einem Zebrafisch mit einem Alter von zumindest 10 Tagen nach der Befruchtung durchgeführt wird, ohne direkte Zufuhr der Testsubstanz zur Umgehung der Blut-Hirn-Schranke, wodurch eine Substanz mit der biologischen Aktivität und der Fähigkeit erhalten wird, die Blut-Hirn-Schranke zu überwinden;
worin sich die angegebenen Alterswerte auf Zebrafische beziehen, die unter Standardbedingungen gezüchtet wurden, und angepasst werden, wenn ein Zebrafisch unter Bedingungen gezüchtet wurde, die das Erreichen eines äquivalenten Entwicklungsstadiums beschleunigen oder verzögern.

4. Verfahren zum Erhalt einer Substanz mit biologischer Aktivität im Zentralnervensystem (ZNS), Hirn oder Auge, wobei das Verfahren einen Screening-Test umfasst, der durch Verabreichung einer Testsubstanz an einen Testzebrafisch durchgeführt wird, wodurch eine Substanz mit dieser biologischen Aktivität erhalten wird, worin der Aufbau des Screening-Tests einen Algorithmus umfasst, der so formuliert ist, dass er das Vorhandensein und den Entstehungszeitpunkt einer Blut-Hirn-Schranke im Zebrafisch berücksichtigt.

5. Verfahren nach Anspruch 4, worin der Algorithmus weiters so formuliert ist, dass er berücksichtigt, ob die Testsubstanz die Blut-Hirn-Schranke überwindet oder nicht.

6. Screening-Verfahren für eine Substanz mit biologischer Aktivität im Zentralnervensystem (ZNS), Hirn oder Auge, das im Wesentlichen wie in Fig. 1 dargelegt aufgebaut ist.

7. Nutzung des Vorhandenseins und des Entstehungszeitpunkts einer Blut-Hirn-Schranke in einem Zebrafisch beim Entwerfen eines Screening-Tests für eine Substanz mit biologischer Aktivität im Zentralnervensystem (ZNS), Hirn oder Auge.

8. Screening-Verfahren zum Erhalt einer Substanz mit biologischer Aktivität, welche die Blut-Hirn-Schranke nicht überwindet, wobei das Verfahren Folgendes umfasst:
das Verabreichen einer Testsubstanz an einen Testzebrafisch,
das Bestimmen der Wirkung der Testsubstanz auf eine Aktivität oder Funktion im Zebrafisch oder der Wirkung der Testsubstanz auf ein Symptom oder eine Erkrankung oder Störung im Testfisch,
wodurch eine Substanz mit dieser biologischen Aktivität identifiziert wird;
worin:
das Verfahren weiters das Bestimmen der Fähigkeit oder Unfähigkeit der Substanz mit dieser biologische Aktivität umfasst, die Blut-Hirn-Schranke im Testzebrafisch mit einem Alter von zumindest 5 Tagen nach der Befruchtung für Moleküle mit 960 Dalton oder mehr oder zumindest 10 Tagen nach der Befruchtung für Moleküle mit weniger als 960 Dalton zu überwinden, wodurch eine Substanz mit dieser biologischen Aktivität, welche die Blut-Hirn-Schranke nicht überwindet, identifiziert wird;
worin sich die angegebenen Alterswerte auf Zebrafische beziehen, die unter Standardbedingungen gezüchtet wurden, und angepasst werden, wenn ein Zebrafisch unter Bedingungen gezüchtet wurde, die das Erreichen eines äquivalenten Entwicklungsstadiums beschleunigen oder verzögern.

9. Verfahren nach Anspruch 8, umfassend das Identifizieren der Substanz mit dieser biologischen Aktivität in einem Testzebrafisch mit einem Alter von weniger als 5 Tagen nach der Befruchtung für ein Molekül mit 960 Dalton oder mehr, weniger als 10 Tagen nach der Befruchtung für ein Molekül mit weniger als 960 Dalton, das nicht durch pgp ausgeschleust wird, oder weniger als 8 Tagen nach der Befruchtung für ein Molekül, das aktiv durch pgp ausgeschleust wird; worin sich die angegebenen Alterswerte auf Zebrafische beziehen, die unter Standardbedingungen gezüchtet wurden, und angepasst werden, wenn ein Zebrafisch unter Bedingungen gezüchtet wurde, die das Erreichen eines äquivalenten Entwicklungsstadiums beschleunigen oder verzögern.

10. Nutzung des Vorhandenseins und des Entstehungszeitpunkts einer Blut-Hirn-Schranke in einem Zebrafisch beim Entwerfen eines Screening-Tests für eine Substanz mit biologischer Aktivität, welche die Blut-Hirn-Schranke nicht überwindet.

11. Verfahren oder Nutzung nach einem der vorangegangenen Ansprüche, das Bestimmen, ob eine Testsubstanz aktiv über die Blut-Hirn-Schranke transportiert wird, umfassend.

12. Verfahren oder Nutzung nach Anspruch 11, das Bestimmen, ob eine Testsubstanz aktiv in das Hirn transportiert wird, umfassend.

13. Verfahren oder Nutzung nach Anspruch 11, das Bestimmen, ob eine Testsubstanz aktiv aus dem Hirn heraustransportiert wird, umfassend.

14. Verfahren oder Nutzung nach einem der vorangegangenen Ansprüche, das Bestimmen, ob eine Testsubstanz durch p-Glykoprotein transportiert wird, umfassend.

15. Verfahren oder Nutzung nach einem der vorangegangenen Ansprüche, das Bestimmen, ob eine Testsubstanz die p-Glykoproteinfunktion stört, umfassend.

16. Verfahren oder Nutzung nach einem der vorangegangenen Ansprüche, das Bestimmen, ob eine Testsubstanz die p-Glykoproteinexpression verändert, umfassend.

17. Verfahren oder Nutzung nach einem der Ansprüche 14 bis 16, worin die Testsubstanz sowohl vor als auch nach der Entwicklung von funktionellen p-Glykoproteinen beurteilt wird.

18. Verfahren oder Nutzung nach Anspruch 17, worin die Testsubstanz am Tag 8 oder 9 nach der Befruchtung sowie am Tag 10 oder danach beurteilt wird, worin sich die angegebenen Alterswerte auf Zebrafische beziehen, die unter Standardbedingungen gezüchtet wurden, und angepasst werden, wenn ein Zebrafisch unter Bedingungen gezüchtet wurde, die das Erreichen eines äquivalenten Entwicklungsstadiums beschleunigen oder verzögern.

19. Verfahren oder Nutzung nach einem der vorangegangenen Ansprüche, das Bestimmen, ob eine Kombination aus Testsubstanzen im Vergleich zu einer isoliert verabreichten Testsubstanz eine veränderte BBB-Überwindung aufweist, umfassend.

20. Verfahren oder Nutzung nach einem der vorangegangenen Ansprüche, das Bestimmen von Körper:Hirn-Arzneimittelkonzentrationsgradienten umfassend.

21. Verfahren oder Nutzung nach einem der vorangegangenen Ansprüche, das Vergleichen der relativen Wirksamkeiten von Testsubstanzen mit gewünschter Aktivität im Zentralnervensystem bei Vorhandensein einer Blut-Hirn-Schranke umfassend.

22. Verfahren oder Nutzung nach einem der vorangegangenen Ansprüche, das Bestimmen der Überwindung der Blut-Retina-Schranke durch eine Testsubstanz umfassend.

23. Verfahren oder Nutzung nach einem der vorangegangenen Ansprüche, das Bestimmen, ob eine Testsubstanz durch die Blut-Hirn-Schranke metabolisiert wird, umfassend.

24. Verwendung eines Zebrafisches zur Bestimmung, ob eine Testsbustanz aktiv über die Blut-Hirn-Schranke transportiert wird.

25. Verwendung eines Zebrafisches zur Bestimmung, ob eine Testsubstanz aktiv in das Hirn transportiert wird.

26. Verwendung eines Zebrafisches zur Bestimmung, ob eine Testsubstanz aktiv aus dem Hirn heraustransportiert wird.

27. Verwendung eines Zebrafischs zur Bestimmung, ob eine Testsubstanz durch g-Glykoprotein über die Blut-Hirn-Schranke transportiert wird.

28. Verwendung eines Zebrafischs zur Bestimmung, ob eine Testsubstanz die p-Glykoproteinfunktion stört.

29. Verwendung eines Zebrafischs zur Bestimmung, ob eine Testsubstanz die p-Glykoproteinexpression verändert.

30. Verwendung nach einem der Ansprüche 27 bis 29, worin die Testsubstanz sowohl vor als auch nach der Entwicklung von funktionellen p-Glykoproteinen beurteilt wird.

31. Verwendung nach Anspruch 30, worin die Testsubstanz am Tag 8 oder 9 nach der Befruchtung sowie am Tag 10 oder danach beurteilt wird, worin sich die angegebenen Alterswerte auf Zebrafische beziehen, die unter Standardbedingungen gezüchtet wurden, und angepasst werden, wenn ein Zebrafisch unter Bedingungen gezüchtet wurde, die das Erreichen eines äquivalenten Entwicklungsstadiums beschleunigen oder verzögern.

32. Verwendung eines Zebrafischs zur Bestimmung, ob eine Kombination aus Testsubstanzen im Vergleich zu einer isoliert verabreichten Testsubstanz eine veränderte BBB-Überwindung aufweist.

33. Verwendung eines Zebrafischs zur Bestimmung von Körper:Hirn-Arzneimittelkonzentrationsgradienten.

34. Verwendung eines Zebrafischs zum Vergleich der relativen Wirksamkeiten von Testsubstanzen mit gewünschter ZNS-Aktivität bei Vorhandensein einer Blut-Hirn-Schranke.

35. Verwendung eines Zebrafischs zur Bestimmung des Überwindens der Blut-Retina-Schranke durch eine Testsubstanz.

36. Verwendung eines Zebrafischs zur Bestimmung, ob eine Testsubstanz durch die Blut-Hirn-Schranke metabolisiert wird.

## Revendications

1. Méthode de criblage pour obtenir une substance ayant une activité biologique sur le système nerveux central (SNC), le cerveau ou l'oeil, la méthode comprenant :
l'administration d'une substance de test à un poisson zèbre de test
la détermination de l'effet de la substance de test sur une activité ou une fonction du système nerveux central, du cerveau ou de l'oeil ou l'effet de la substance de test sur un symptôme ou une maladie ou un trouble du système nerveux central, du cerveau ou de l'oeil du poisson de test
pour ainsi identifier une substance ayant ladite activité biologique ; où
la substance de test est connue pour pénétrer la barrière sang-cerveau et la méthode est accomplie sur un poisson zèbre de tout âge ;
ou bien la substance de test est connue pour ne pas traverser la barrière sang-cerveau et la méthode est accomplie sur un poisson zèbre d'un âge inférieur à cinq jours après fertilisation pour des molécules de 960 daltons
ou plus, moins de dix jours après fertilisation pour des molécules de moins de 960 daltons ne subissant pas un efflux par la p-glycoprotéine (pgp) ou moins de huit jours après fertilisation pour des molécules subissant un efflux actif par pgp et/ou est accomplie sur un poisson zèbre d'un âge d'au moins cinq jours après fertilisation pour des molécules de 960 daltons ou plus, ou au moins dix jours après fertilisation pour des molécules de moins de 960 daltons ne subissant pas d'efflux par pgp et au moins huit jours après fertilisation pour des molécules subissant un efflux actif par pgp et comprend la délivrance directe de la substance de test pour contourner la barrière sang-cerveau ;
ou, sans savoir si la substance de test est capable de traverser la barrière sang-cerveau ou non, la méthode est accomplie sur un poisson zèbre d'un âge d'au moins cinq jours après fertilisation pour des molécules de 960 daltons
ou plus et au moins dix jours après fertilisation pour des molécules de moins de 960 daltons, avec délivrance directe de la substance de test pour contourner la barrière sang-cerveau ;
où les âges indiqués se rapportent à un poisson zèbre développé en conditions standards et ils sont ajustés si les poissons zèbres sont développés dans des conditions qui accélèrent ou retardent l'atteinte de l'âge équivalent du développement.

2. Méthode selon la revendication 1, où ladite délivrance directe comprend l'injection dans le cerveau ou l'oeil.

3. Méthode de criblage pour obtenir une substance ayant une activité biologique sur le système nerveux central (SNC), le cerveau ou l'oeil, la méthode comprenant :
l'administration d'une substance de test à un poisson zèbre de test,
la détermination de l'effet de la substance de test sur une activité ou une fonction du système nerveux central, du cerveau ou de l'oeil, ou l'effet de la substance de test sur un symptôme ou une maladie ou un trouble du système nerveux central, du cerveau ou de l'oeil du poisson test,
pour ainsi identifier une substance ayant ladite activité biologique ;
où la méthode est accomplie pour des molécules de 960 daltons ou plus sur un poisson zèbre d'un âge de moins de cinq jours après fertilisation et sur un poisson zèbre d'un âge de moins cinq jours après fertilisation ou est accomplie pour des molécules de moins de 960 daltons sur un poisson zèbre d'un âge de moins de dix jours après fertilisation et sur un poisson zèbre d'un âge d'au moins dix jours après fertilisation, sans délivrance directe de la substance de test pour contourner la barrière sang-cerveau, ainsi une substance qui a ladite activité biologique et a la capacité de traverser la barrière sang-cerveau est obtenue ;
où les âges indiqués se rapportent à un poisson zèbre développé en conditions standards et ils sont ajustés si le poisson zèbre est développé dans des conditions qui accélèrent ou retardent l'obtention du stade équivalent de développement.

4. Méthode pour obtenir une substance ayant une activité biologique sur le système nerveux central (SNC), le cerveau ou l'oeil, la méthode comprenant un essai de criblage entrepris en administrant une substance de test à un poisson zèbre de test pour qu'ainsi une substance ayant ladite activité biologique soit obtenue, où la conception de l'essai de criblage emploie un algorithme formulé pour tenir compte de la présence de et du moment de la formation d'une barrière sang-cerveau chez le poisson zèbre.

5. Méthode selon la revendication 4, où l'algorithme est de plus formulé pour tenir compte du fait que la substance de test traverse ou non la barrière sang-cerveau.

6. Méthode de criblage pour une substance ayant une activité biologique sur le système nerveux central (SNC), le cerveau ou l'oeil, sensiblement telle qu'indiquée à la figure 1.

7. Utilisation de l'existence de et du moment de formation d'une barrière sang-cerveau dans un poisson zèbre dans la conception d'un essai de criblage pour une substance ayant une activité biologique sur le système nerveux central (SNC), le cerveau ou l'oeil.

8. Méthode de criblage pour obtenir une substance ayant une activité biologique, laquelle substance ne traverse pas la barrière sang-cerveau, la méthode comprenant :
l'administration d'une substance de test à un poisson zèbre de test
la détermination de l'effet de la substance de test sur une activité ou une fonction du poisson zèbre ou bien l'effet de la substance de test sur un symptôme ou une maladie ou un trouble du poisson test
pour ainsi identifier une substance ayant ladite activité biologique
où la méthode comprend de plus la détermination de l'aptitude ou de l'incapacité de la substance ayant ladite activité biologique à traverser la barrière sang-cerveau dans le poisson zèbre de test d'un âge d'au moins cinq jours après fertilisation pour des molécules de 960 daltons
ou plus ou d'au moins dix jours après fertilisation pour des molécules de moins de 960 daltons, pour ainsi identifier une substance ayant ladite activité biologique et qui ne traverse pas la barrière sang-cerveau ;
où les âges indiqués se rapportent à un poisson zèbre développé dans des conditions standards et ils sont ajustés si le poisson zèbre est développé dans des conditions qui accélèrent ou retardent l'obtention d'un stade équivalent de développement.

9. Méthode selon la revendication 8, comprenant l'identification de la substance ayant ladite activité biologique chez un poisson zèbre de test d'un âge inférieur à cinq jours après fertilisation pour une molécule de 960 daltons ou plus, de moins de dix jours après fertilisation pour une molécule de moins de 960 daltons, ne subissant pas d'efflux par pgp au moins de huit jours après fertilisation pour une molécule subissant un efflux actif par pgp ; où les âges indiqués se rapportent à un poisson zèbre développé en conditions standards et ils sont ajustés si le poisson zèbre est développé dans des conditions qui accélèrent ou retardent l'obtention du stade équivalent de développement.

10. Utilisation de l'existence de et du moment de formation d'une barrière sang-cerveau chez un poisson zèbre dans la conception d'un essai de criblage pour une substance ayant une activité biologique et qui ne traverse pas la barrière sang-cerveau.

11. Méthode ou utilisation selon l'une quelconque des revendications précédentes, consistant à déterminer si une substance de test est activement transportée à travers la barrière sang-cerveau.

12. Méthode ou utilisation selon la revendication 11, comprenant la détermination du fait qu'une substance de test est activement transportée vers le cerveau.

13. Méthode ou utilisation selon la revendication 11, consistant à déterminer si une substance de test est activement transportée hors du cerveau.

14. Méthode ou utilisation selon l'une quelconque des revendications précédentes, consistant à déterminer si une substance de test est transportée par la p-glycoprotéine.

15. Méthode ou utilisation selon l'une quelconque des revendications précédentes, consistant à déterminer si une substance de test interfère avec la fonction de la p-glycoprotéine.

16. Méthode ou utilisation selon l'une quelconque des revendications précédentes, consistant à déterminer si une substance de test modifie l'expression de la p-glycoprotéine.

17. Méthode ou utilisation selon l'une quelconque des revendications 14 à 16, où la substance de test est évaluée à la fois avant et après développement de p-glycoprotéines fonctionnelles.

18. Méthode ou utilisation selon la revendication 17, où la substance de test est évaluée au jour 8 ou 9 après fertilisation ainsi qu'au jour 10 ou après, où les âges indiqués se rapportent à un poisson zèbre développé en conditions standards et ils sont ajustés si le poisson zèbre est développé dans des conditions qui accélèrent ou retardent l'obtention du stade équivalent de développement.

19. Méthode ou utilisation selon l'une quelconque des revendications précédentes, consistant à déterminer si une combinaison de substances de test a une pénétration modifiée de la barrière sang-cerveau en comparaison avec une substance de test donnée de façon isolée.

20. Méthode ou utilisation selon l'une quelconque des revendications précédentes, comprenant la détermination de gradients de concentrations de produits pharmaceutiques corps : cerveau.

21. Méthode ou utilisation selon l'une quelconque des revendications précédentes, comprenant la comparaison des efficacités relatives des substances de test avec l'activité souhaitée dans le système nerveux central en présence d'une barrière sang-cerveau.

22. Méthode ou utilisation selon l'une quelconque des revendications précédentes, comprenant la détermination de la pénétration d'une substance de test à travers la barrière sang-rétine.

23. Méthode ou utilisation selon l'une quelconque des revendications précédentes, consistant à déterminer si une substance de test est métabolisée par la barrière sang-cerveau.

24. Utilisation d'un poisson zèbre pour déterminer si une substance de test est activement transportée à travers la barrière sang-cerveau.

25. Utilisation d'un poisson zèbre pour déterminer si une substance de test est activement transportée vers le cerveau.

26. Utilisation d'un poisson zèbre pour déterminer si une substance de test est activement transportée hors du cerveau.

27. Utilisation d'un poisson zèbre pour déterminer si une substance de test est transportée à travers la barrière sang-cerveau par la p-glycoprotéine.

28. Utilisation d'un poisson zèbre pour déterminer si une substance de test interfère avec la fonction de la p-glycoprotéine.

29. Utilisation d'un poisson zèbre pour déterminer si une substance de test modifie l'expression de la p-glycoprotéine.

30. Utilisation selon l'une quelconque des revendications 27 à 29, où la substance de test est évaluée avant et après développement de p-glycoprotéines fonctionnelles.

31. Utilisation selon la revendication 30, où la substance de test est évaluée au jour 8 ou 9 après fertilisation ainsi qu'au jour 10 ou après, où les âges indiqués se rapportent à un poisson zèbre développé en conditions standards et sont ajustés si le poisson zèbre est développé en conditions qui accélèrent ou retardent l'obtention du stade équivalent de développement.

32. Utilisation d'un poisson zèbre pour déterminer si une combinaison de substances de test a modifié la pénétration de la barrière sang-cerveau en comparaison avec une substance de test donnée de façon isolée.

33. Utilisation d'un poisson zèbre pour déterminer les gradients de concentrations de produits pharmaceutiques corps : cerveau.

34. Utilisation d'un poisson zèbre pour comparer les efficacités relatives de substances de test ayant une activité souhaitée sur SNC en présence d'une barrière sang-cerveau.

35. Utilisation d'un poisson zèbre pour déterminer la pénétration d'une substance de test à travers la barrière sang-rétine.

36. Utilisation d'un poisson zèbre pour déterminer si une substance de test est métabolisée par la barrière sang-cerveau.
